# EUROPEAN PATENT SPECIFICATION

(11) **EP 0 425 821 B1**
(45) Date of publication and mention of the grant of the patent: **10.08.1994**
(21) Application number: 90118695.7
(22) Date of filing: 28.09.1990
(51) Int. Cl.: C07K 13/00, A61K 37/02

(54) **The use of human ADF (=Adult T-cell leukemia-derived factor) for producing a medicament**
Verwendung von Human-ADF (=Adult T-cell leukemia-derived factor) zur Herstellung von Medikamenten
L'utilisation d'ADF humain (=Adult T-cell leukemia-derived factor) pour la fabrication de médicaments

(30) Priority: 29.09.1989 JP 256369/89
(43) Date of publication of application: 08.05.1991
(73) Proprietor: AJINOMOTO CO., INC., Tokyo 104 (JP); Yodoi, Junji, Sakyo-ku Kyoto-Shi Kyoto-fu (JP)
(72) Inventor: Yodoi, Junji, Kyoto-ku, Kyoto-shi, Sakyo-ku (JP); Uchida, Atsushi, Kyoto-fu, Uji-shi (JP); Tagaya, Yutaka, Rokko Takaha Urban Life 107, Nada-ku, Takaha-aza (JP); Mitsui, Akira, c/o Central Research Laboratories, Kawasaki-ku, Kawasaki-shi, Kanagawa-ken (JP); Hirakawa, Tadashi, c/o Central Research Lab., Kawasaki-ku, Kawasaki-shi, Kanagawa-ken (JP)
(74) Representative: Strehl Schübel-Hopf Groening & Partner

(56) References cited:
- EP-A- 0 237 189
- EP-A- 0 299 206
- BIOCHEMICAL AND BIOPHYSICAL RESEARCH COMMUNICATIONS, vol. 136, no. 2, 29thApril 1986, pages 630-637, Duluth, US; K.U. SCHALLREUTER et al.: "The role of
- thioredoxin reductase in the reduction of free radicals at the surface of the epidermis"
- EXPERIENTIA, vol. 45, January 1989, pages 41-52, Basel, CH; A. ROJAS et al.:"Modifiers of radiosensitivity"
- BIOCHEMICAL AND BIOPHYSICAL RESEARCH COMMUNICATIONS, vol. 163, no. 3, 29th September 1989, pages 1466-1472, Duluth, US; M. KUZUYA et al.: "Protective role of intracellular glutathione against oxidized low density lipoprotein incultured endothelial cells"
- RHEUMATOL. INT., vol. 4, 1984, pages 35-38, Berlin, DE; N.D. HALL et al.: "The oxidation of serum sulph-hydryl groups by hydrogen peroxide secreted by stimulated phagocytic cells in rheumatoid arthritis"

## Description

The present invention relates to the use of human ADF for producing a new medicament.
Human adult T cell leukemia-derived factor (human ADF) is a substance having cell differentiation and growth induction activity for blood cells, such as lymphocytes and fibroplasts and is useful as a therapeutic agent for cancer and diseases associated with immunodeficiency.
According to a publication of Wollman et al. in the Journal of Biological Chemistry, Vol.263 (No. 30), PP. 15506-15512, 1988) thioredoxin, which is an oxidation-reduction protein in the human body has an amino acid sequence, which is similar to that of human ADF with the exception of two different amino acids.
Human ADF also shows oxidation-reduction activity similar to thioredoxin. Therefore some researchers referred to human ADF as thioredoxin; in the present description however the term human ADF is used in the following.

The present invention relates to a radioprotective composition, an antiinflammatory composition, a composition for the treatment of rheumatism, a composition for the treatment of autoimmune diseases, a composition for the treatment of ischemic damages of organs, a composition for the treatment of drug toxicity, and a composition for the treatment of arteriosclerosis, comprising as an effective ingredient human adult T cell leukemia-derived factor (hereafter referred to as human ADF). By the use of human ADF in accordance with the present invention in combination with radiotherapy for cancer, etc., side effects caused by radiation can be greatly reduced. Furthermore, human ADF has an activity of neutralizing free radicals and hence, can be widely utilized as an agent for the treatment or prevention of various diseases which are accompanied by tissue damages due to peroxidation caused by free radicals in the living body, such as inflammation, rheumatism, autoimmune disease, ischemic damages of organs, drug toxicity, arteriosclerosis, etc.. Radiation is one of the most effective means for the therapy of cancer. However, radiation non-specifically destroys not only cancer cells but also normal cells. Consequently when the dose of radiation is increased for the purpose of enhancing its therapeutic effect, side effects such as erythropoietic disorders, pyrexia, vomiting, etc. are unavoidable. Furthermore, even though cancer is cured, sequelae such as developmental anomaly due to abnormalities in internal secretion, disorders of the central nervous system, etc. occur and pose problems. In the actual therapy, attention has been paid only to the tumor regression effect. However, as therapeutic results are improved and life span increases, it has become essential to ensure the "quality of life" of pre- and post-operative patients or patients with increased life span.
It is thus an important idea to minimize the disturbance of normal cells as far as possible and to suppress side effects by using radiation protecting agents in combination with radiotherapy to enhance the therapeutic results.
Cytotoxicity due to radiation is considered to be caused by free radicals produced by radiation in living bodies. Based on the concept of inactivating the free radicals to alleviate the side effects, reducing glutathione (GSH) or other SH compounds have been investigated and developed as radiation protecting agents. GSH was recognized to be effective in vitro. However since it cannot permeate through cell membranes, its effect is hardly noted when GSH is administered to an animal. Furthermore, WR-2721 (S-2-(3-aminopropyl-amino)-ethylphosphorothioic acid) which is one of SH compounds which were developed in the United States of America could prevent disorders of bone marrow stem cells caused by radiation.
However, WR-2721 itself has serious side effects, and was thus abandonned. On the other hand, it was found that interleukin 1 (hereafter simply referred to as IL1) which is a protein derived from the living body also has a radiation protecting activity. However, IL1 is pyretic and its dose is thus limited when IL1 is administered to human.
In addition, its mechanism is not exactly known so that it is difficult to freely control its activity. Therefore, a protecting agent which can effectively prevent side effects in radiotherapy and which itself has low toxicity has not yet been found. Free radicals which are considered to be a cause for the injury of the body caused by radiation are generated in the body in large quantities also in inflammation, rheumatism, autoimmune diseases, ischemic damages of organs, intoxication by drugs, etc. It is considered that free radicals may attack plasma membranes, proteins, enzymes, or DNAs through their potent oxidation (peroxidation) activity to injure the living body.
Arteriosclerosis is also considered to be caused by accumulation of lipid peroxide which is one of those free radicals. It is therefore considered that SH compounds having a radioprotective activity should have a strong free radical scavenging action, and that these compounds can be effectively utilized as therapeutic or prophylactic agents for the treatment of inflammation or the other various diseases described above which are associated with peroxidation in the body. On the other hand, superoxide dismutase (SOD) has an activity of scavenging O$\frac{\text{-}}{\text{2}}$ which is one of the free radicals. Investigations are now under way to develop the use of SOD as an antiinflammatory agent. However, its half-life in the body is as short as 10 minutes so that it is necessary to carry out chemical modification or to include the substance in chemical modification or to include the substance in liposomes, etc., in order to prolong the half-life period. Such necessity results in problems in the clinical application.
The free radical scavening activity of human ADF (human thioredoxin) having an oxidation-reduction ability has not been elucidated until the present invention was made. An object of the present invention is to provide pharmaceutical compositions which can effectively reduce the side effects caused by radiation in radiotherapy of cancer, etc., and furthermore can be effective for the treatment and prevention of (1) inflammation caused by free radicals or (2) various inflammation associated with peroxidation in the body, and which have low toxicity.
As a result of extensive investigations to solve the problems described above, it has been found that human ADF has excellent radioprotective and antiinflammatory effects and excellent effects of treating rheumatism, autoimmune diseases, ischemic damage of organs, drug toxicity and arteriosclerosis.

The present invention relates to the use of human ADF for producing a radioprotective composition.
The present invention furthermore relates to the use of human ADF as an effective ingredient for producing an antiinflammatory composition, a composition for the treatment of rheumatism, a composition for the treatment of autoimmune diseases, a composition for the treatment of ischemic damage of organs, a composition for the treatment of drug toxicity and a composition for the treatment of arteriosclerosis.

Hereafter the present invention is described in detail. Human ADF is a protein firstly found in the culture supernatant of T cell leukemia cell line (ATL-2) established from a patient with human adult T cell leukemia. The present inventor has already succeeded in the purification of the protein and cloning of cDNA and also suceeded in mass production of recombinant human ADF by genetic recombination (Japanese Patent Application Laid-Open No. 85097/1989 and EP-A-299 206). Human ADF has a structure of active site common to thioredoxin, the redox protein derived from Escherichia coli, higher plants, rabbits, etc.
In addition, it has also be confirmed that recombinant human ADF has thioredoxin-like reducing activity.

Human ADF used according to the present invention is a polypeptide having any of the following amino acid sequences:
(a) 
1
Val-Lys-Gln-Ile-Glu-Ser-Lys-Thr-Ala-Phe-Gln-

Glu-Ala-Leu-Asp-Ala-Ala-Gly-Asp-Lys-Leu-Val-

Val-Val-Asp-Phe-Ser-Ala-Thr-Trp-Cys-Gly-Pro-

Cys-Lys-Met-Ile-Lys-Pro-Phe-Phe-His-Ser-Leu-
50
Ser-Glu-Lys-Tyr-Ser-Asn-Val-Ile-Phe-Leu-Glu-

Val-Asp-Val-Asp-Asp-Cys-Gln-Asp-Val-Ala-Ser-

Glu-Cys-Glu-Val-Lys-Cys-Met-Pro-Thr-Phe-Gln-

Phe-Phe-Lys-Lys-Gly-Gln-Lys-Val-Gly-Glu-Phe-

Ser-Gly-Ala-Asn-Lys-Glu-Lys-Leu-Glu-Ala-Thr-
100
Ile-Asn-Glu-Leu-Val
(b) a polypeptide which in respect to (a) is deficient in one or more amino acids;
(c) a polypeptide in which in respect to (a) one or more amino acids are replaced;
(d) a fusion polypeptide comprising a polypeptide according to (a), (b) or (c) and additional amino acids in which the additional connected amino acids to not interfere with the biological ADF activity or may be easily eliminated;
(e) a polypeptide which is an allelic derivative of a polypeptide according to any of (a), (b), (c) or d)
(f) a polypeptide, in which in the amino acid sequence (a) or (b) additional amino acids are inserted, and
(g) a polypeptide having any of the amino acid sequences (a) to (f) which is chemically modified or to which sugar chains are linked.

The human ADF protein used in the present invention may be prepared by the following method or any other methods, as long as the product obtained has the stated activity. Therefore the method of producing ADF is not limited.
(1) Human ADF protein is purified from the culture supernatant of cells or cell extract of human-derived cell line (ATL-2, etc.) in a conventional manner such as salting out, gel filtration chromatography, ion exchange chromatography, affinity chromatography, chromatofocusing, reverse phase chromatography, hydrophobic chromatography, etc. (Japanese Patent Application Laid-Open No. 85097/1989).
(2) By genetic recombination technique, cDNA or genome DNA of human ADF is transduced into host cells such as Escherichia coli, Bacillus subtilis, yeast, higher animal cells, plant cells, etc. and recombinant human ADF protein expressed in the host cells is purified by means as described in (1) (Japanese Patent Application Laid-Open No. 85097/1989, or EP-A-299 206).
(3) By chemical peptide synthesis, a polypeptide having the sequence (I) is synthesized.

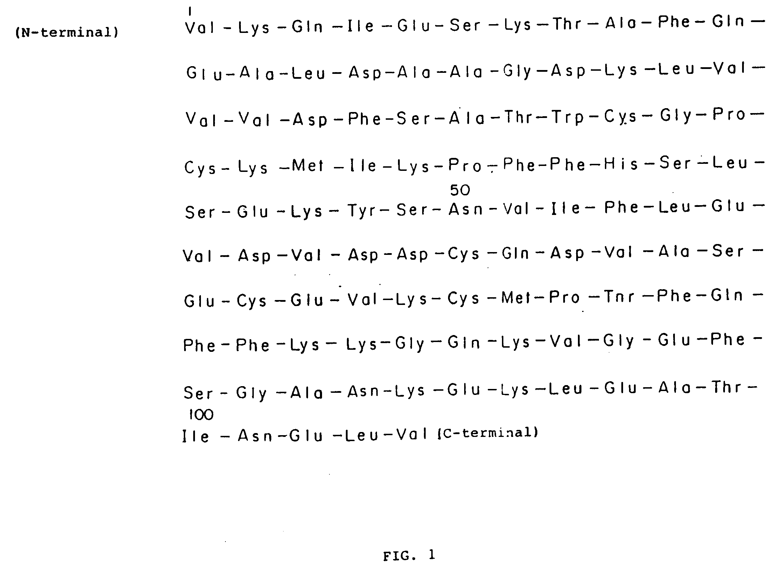

Any of the methods described above may be used.
By administering a composition produced by the use of human ADF according to the present invention upon radiotherapy, side effects caused by radiation can be greatly reduced. For example, human ADF can be applied to the treatment of various diseases including various cancers requiring treatment by total body or local irradiation with X rays and to various diseases, such as acute and chronic leukemia and aplastic anemia, which require total body irradiation with X rays prior to transplantation of bone marrow. Since human ADF is a protein derived from the human body it is not recognized as a foreign matter when it is administered to the human body and its toxicity is thus very low. Human ADF is administered in a dose of 1 to 30 mg/kg body weight before or after or, before and after irradiation by dividing it into several portions. The period for administration is desirably within one day immediately before or immediately after irradiation. The dose is preferably about 10 mg/kg body weight but may be varied depending upon dose of radiation and the condition of the patient. The route for the administration may be intravenous administration, intramuscular administration or any other administration. The present invention as taught by human ADF is also capable of reducing and scavenging various free radicals. When free radicals react with proteins or enzymes having S-S crosslinks, an incorrect S-S linkage is formed intramolecularly or intermolecularly to destroy their activity.
The human ADF used according to the present invention also has the ability of correcting the S-S crosslinking in the thus inactivated proteins or enzymes and of restoring its activity.
Therefore, the conditions in inflammation accompanied by injury of the body due to free radicals or diseases such as rheumatism, autoimmune disease, ischemic damage of organs and drug toxicity, etc. can be greatly alleviated by administering the human ADF. The human ADF can also be utilized as an agent for the prevention and treatment of arteriosclerosis which is considered to be caused by accumulation of free radicals. Again, the composition comprising the human ADF may also be utilized over a wide range as an agent for the prevention and treatment of rheumatism, autoimmune disease, ischemic damage of organs, chemical toxication, arteriosclerosis and the like in addition to its use as a radiation protecting agent or an antiinflammatory agent.
The human ADF used in the present invention is not limited to the polypeptide having the sequence shown in Fig. 1.
That is, a polypeptide added with methionine residue at the N-terminal thereof, a polypeptide having an amino acid sequence with additional substitution by chemical modification or nucleotide substitution, a polypeptide having an amino acid sequence which is deficient of one or more amino acids, a polypeptide having an amino acid sequence with insertion, or a polypeptide added with sugar chain, etc. at the side chain thereof may also be used, as long as they retain the human ADF activity. Preferred polypeptides are a polypeptide having the amino acid sequence shown in Fig. 1 and a polypeptide having a structure in which Met is added to the N-terminal of the amino acid sequence shown in Fig. 1. Furthermore, since the active site of human ADF has already been revealed it is also possible to freely control its activity by means of protein engineering.
The content of the human ADF in the pharmaceutical composition of the present invention comprising the human ADF is not particularly limited. In any case, however, the human ADF may be contained in a dose of 0.01 to 100.0 wt%, preferably 0.1 to 50 wt%. In addition to the human ADF, various stabilizers and excipients such as mannitol, maltose, etc. may be added.

### Brief Description of the Drawings

Fig. 1 shows the amino acid sequence of human ADF.

Fig. 2 shows the radioprotective effect of human ADF.

Fig. 3 shows the reducing activity of free radicals by human ADF.

Fig. 4 shows the reactivation effect of inactivated enzyme by human ADF.

Fig. 5 shows the structural change in RNase.

Fig. 6 shows the kinetics of human ADF in blood.

Hereafter the present invention is described with reference to the examples.

### Example 1

### Preparation of recombinant human ADF.

Following (Japanese Patent Application Laid-Open No. 85077/1989), recombinant human ADF was prepared. Firstly, Escherichia coli was transformed with plasmid DNA into which human ADF cDNA had been incorporated, to express human ADF in Escherichia coli. Subsequent purification by ion exchange chromatography etc. gave recombinant human ADF. From 20 liters of the culture of Escherichia coli, 1 g of the purified protein was obtained. The standard product showed a single band of molecular weight of 12,000 by SDS-PAGE. By immunoblotting, it reacted with anti-human ADF antibodies. The thus obtained human ADF had a reducing activity as thioredoxin. Its specific activity was comparable to that of Escherichia coli-derived thioredoxin. After this recombinant human ADF was dialyzed to phosphate buffered saline (PBS) overnight, the dialysate was passed through a millipore filter of 0.22 µm to sterilize it.
After adjusting its concentration to 0.5 mg/ml, the recombinant human ADF was used for the following experiment.

### Example 2

### Radioprotective effect of recombinant human ADF in vivo.

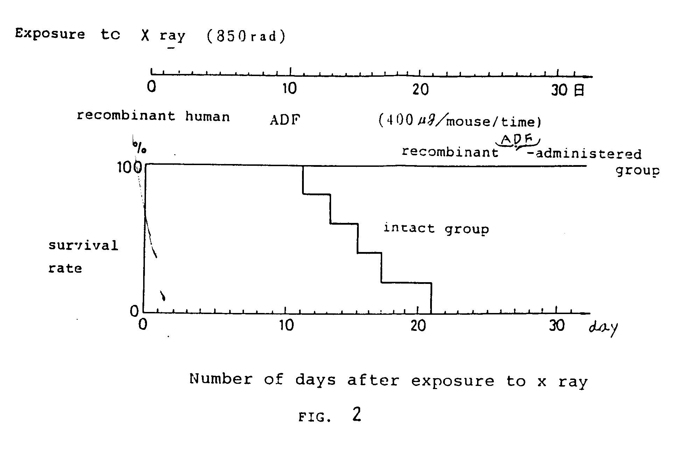

Immediately after (Day 0) irradiation with X rays in 8.5 Gy to mice (ICR, age of 9 weeks, male), recombinant human ADF was intraperitoneally administered every other day for 10 days by 6 times in total. The dose was 400 µg/mouse/time.
As shown in Fig. 2, mice in the control group to which no human ADF was administered began to die on or after Day 11 from the irradiation and all mice died up to the Day 21. To the contrary, all mice survived even on Day 30 in the recombinant human ADF-administered group.

### Example 3

### Reducing activity of free radical by recombinant human ADF.

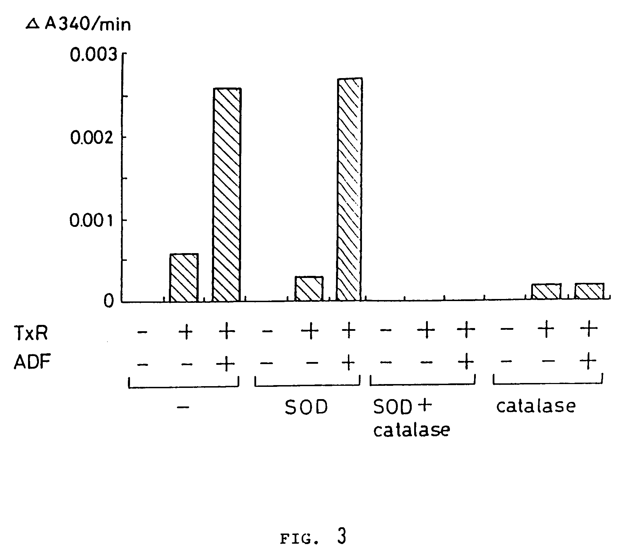

It was revealed that the human ADF of the present invention had an activity of reducing free radicals. As shown in Fig. 3, the system of forming free radicals (O$\frac{\text{-}}{\text{2}}$, H₂O₂) consisting of xanthine oxidase and xanthine was added to the reduced thioredoxin reproduction system consisting of recombinant human ADF, thioredoxin reductase (TxR) and NADPH, whereby reduction in absorbance at 340 nm associated with consumption of NADPH was observed. This reaction was hardly observed in the absence of ADF. Furthermore, this reaction was not inhibited by SOD which was a superoxide (O$\frac{\text{-}}{\text{2}}$) scavenging enzyme but inhibited by catalase which was a hydrogen peroxide (H₂O₂) scavenging enzyme. It was thus revealed that the human ADF was capable of reducing hydrogen peroxide.
In Fig. 3, Δ A340/min shows a decrease in absorbance at 340 nm.

### Example 4

### Reactivation effect of inactivated enzyme by recombinant human ADF.

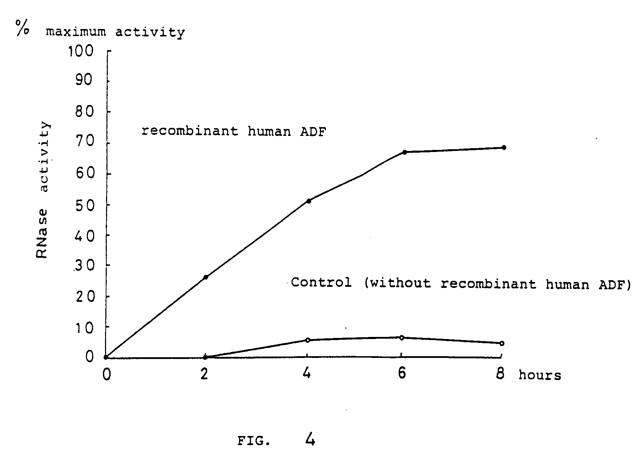

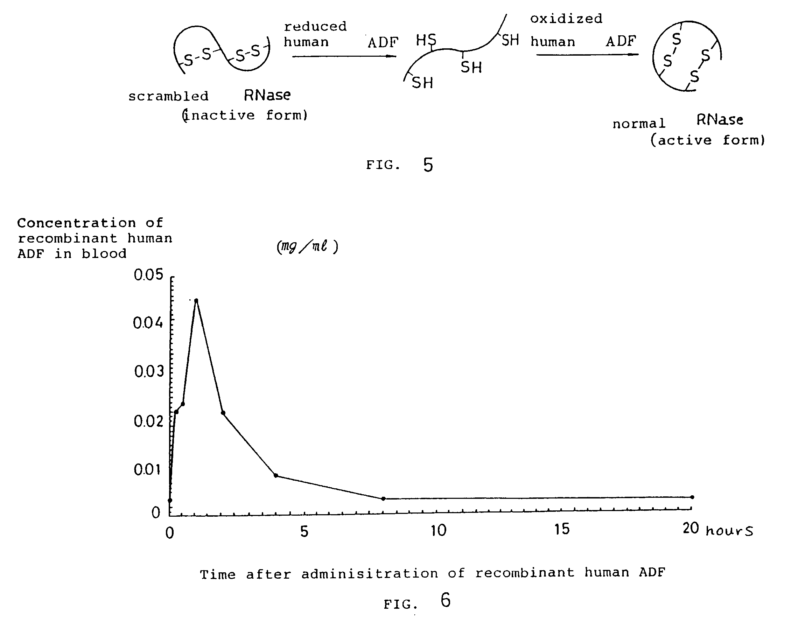

It was revealed that the human ADF of the present invention had an activity of restoring the activity of an enzyme inactivated by free radicals (Fig. 4). Ribonuclease (RNase) possesses four S-S linkages in the molecule thereof but when the S-S linkages are reduced with a reducing agent, dithiothreitol (DTT), followed by reacting with hydrogen peroxide (H₂O₂) which is one of free radicals , incorrect S-S crosslinkages are formed intramolecularly or intermolecularly, with the result that scrambled RNase without activity was formed. The scrambled RNase has lost its activity, but when the oxidized and reduced recombinant human ADF was added at this stage, the activity of RNase was recovered with passage of time. As shown in Fig. 5, this reaction is considered to be based on the fact that the incorrect S-S linkages would be cleaved by the reduced ADF and the correct S-S linkages would then be formed by the oxidized ADF. The RNase activity was determined by measuring the rate of increasing absorbance at 286 nm associated with hydrolysis of 2′, 3′-cCMP.
The results are shown in Fig. 4.

### Example 5

### Kinetics of recombinant human ADF in blood.

After 2.5 mg (dissolved in 1 ml of physiological saline) of recombinant human ADF (hereafter simply referred to as rADF) was intraperitoneally administered to mouse (C57BL/6, age of 4 weeks, female), the mouse was anesthesized with ether after a definite period of time had lapsed. Blood was then collected from the heart. After that, the serum was collected. After rADF contained in the sera was detected by SDS-PAGE and immunoblotting, bands of rADF on the blotting membrane were quantitatively determined by using densitometer to calculate the concentration of rADF in serum. As shown in Fig. 6, rADF appeared in the blood at 15 minutes after the administration. The blood concentration reached the maximum at 1 hr after administration and then gradually decreased. From the results, the half-life of rADF in blood was calculated to be about 1.5 hour.
The results indicate that the half-life of rADF in blood was obviously longer than that of SOD.

### Effects of the Invention

The human ADF used according to the present invention is a useful substance having an excellent radioprotective effect and in addition thereto, having an excellent activity of scavenging free radicals and an activity of reactivating enzymes inactivated by free radicals. Accordingly, the pharmaceutical composition comprising the human ADF as an effective ingredient is not only useful as a radioprotective agent but also as an agent for the treatment of inflammation accompanied by disturbance of the body due to free radicals, or as an agent for the treatment and prevention of rheumatism, autoimmune disease, ischemic damage of organs, drug toxicity and arteriosclerosis, etc.
Furthermore, the human ADF is a protein derived from human and hence, is not recognized as a foreign matter when it is administered to human beings so that its toxicity is extremely low. In addition, since the half-life in blood is as extremely long such as 1.5 hour and is longer by more than 10 times that of SOD, the human ADF is also characterized in that the human ADF exhibits its effects in a much lower concentration than in SOD.

## Claims

1. The use of a polypeptide having human ADF activity, selected from any of the following polypeptides (a) to (g)
(a) 
1
Val-Lys-Gln-Ile-Glu-Ser-Lys-Thr-Ala-Phe-Gln-
Glu-Ala-Leu-Asp-Ala-Ala-Gly-Asp-Lys-Leu-Val-
Val-Val-Asp-Phe-Ser-Ala-Thr-Trp-Cys-Gly-Pro-
Cys-Lys-Met-Ile-Lys-Pro-Phe-Phe-His-Ser-Leu-
50
Ser-Glu-Lys-Tyr-Ser-Asn-Val-Ile-Phe-Leu-Glu-
Val-Asp-Val-Asp-Asp-Cys-Gln-Asp-Val-Ala-Ser-
Glu-Cys-Glu-Val-Lys-Cys-Met-Pro-Thr-Phe-Gln-
Phe-Phe-Lys-Lys-Gly-Gln-Lys-Val-Gly-Glu-Phe-
Ser-Gly-Ala-Asn-Lys-Glu-Lys-Leu-Glu-Ala-Thr-
100
Ile-Asn-Glu-Leu-Val ;
(b) a polypeptide which in respect to (a) is deficient in one or more amino acids;
(c) a polypeptide in which in respect to (a) one or more amino acids are replaced;
(d) a fusion polypeptide comprising a polypeptide according to (a), (b) or (c) and additional amino acids in which the additional connected amino acids do not interfere with the biological ADF activity or may be easily eliminated;
(e) a polypeptide which is an allelic derivative of a polypeptide according to any of (a), (b), (c) or d)
(f) a polypeptide, in which in the amino acid sequence (a) or (b) additional amino acids are inserted, and
(g) a polypeptide having any of the amino acid sequences (a) to (f) which is chemically modified or to which sugar chains are linked,
as an effective ingredient for preparing a radioprotective composition.

2. The use of a polypeptide having human ADF activity as defined in claim (1) as an effective ingredient for preparing an antiinflammatory composition.

3. The use of a polypeptide having human ADF activity as defined in claim (1) as an effective ingredient for preparing a composition for the treatment of rheumatism.

4. The use of a polypeptide having human ADF activity as defined in claim (1) as an effective ingredient for preparing a composition for the treatment of autoimmune diseases.

5. The use of a polypeptide having human ADF activity as defined in claim (1) as an effective ingredient for preparing a composition for the treatment of ischemic damage of organs.

6. The use of a polypeptide having human ADF activity as defined in claim (1) as an effective ingredient for preparing a composition for the treatment of drug toxicity.

7. The use of a polypeptide having human ADF activity as defined in claim (1) as an effective ingredient for preparing a composition for the treatment of arteriosclerosis.

8. The use according to any of the claims 1 to 7 wherein the human ADF used as an effective ingredient is produced in Escherichia coli.

## Patentansprüche

1. Verwendung eines Polypeptids mit humaner ADF-Aktivität, das ausgewählt ist unter den folgenden Polypeptiden (a) bis (g)
(a) 
1
Val-Lys-Gln-Ile-Glu-Ser-Lys-Thr-Ala-Phe-Gln-
Glu-Ala-Leu-Asp-Ala-Ala-Gly-Asp-Lys-Leu-Val-
Val-Val-Asp-Phe-Ser-Ala-Thr-Trp-Cys-Gly-Pro-
Cys-Lys-Met-Ile-Lys-Pro-Phe-Phe-His-Ser-Leu-
50
Ser-Glu-Lys-Tyr-Ser-Asn-Val-Ile-Phe-Leu-Glu-
Val-Asp-Val-Asp-Asp-Cys-Gln-Asp-Val-Ala-Ser-
Glu-Cys-Glu-Val-Lys-Cys-Met-Pro-Thr-Phe-Gln-
Phe-Phe-Lys-Lys-Gly-Gln-Lys-Val-Gly-Glu-Phe-
Ser-Gly-Ala-Asn-Lys-Glu-Lys-Leu-Glu-Ala-Thr-
100
Ile-Asn-Glu-Leu-Val.;
(b) einem Polypeptid, dem bezüglich (a) eine oder mehrere Aminosäuren fehlen;
(c) einem Polypeptid, in dem bezüglich (a) ein oder mehrere Aminosäuren ersetzt sind;
(d) einem Fusionspolypeptid, welches ein Polypeptid gemäß (a), (b) oder (c) und zusätzliche Aminosäuren enthält, wobei die zusätzlich hinzugefügten Aminosäuren die biologische ADF-Aktivität nicht beeinträchtigen oder leicht entfernbar sind;
(e) einem Polypeptid, das eine allele Variante eines Polypeptids nach (a), (b), (c) oder (d) darstellt;
(f) einem Polypeptid, bei dem in die Aminosäuresequenz (a) oder (b) zusätzliche Aminosäuren inseriert sind, und
(g) einem Polypeptid mit einer der Sequenzen (a) bis (f), das chemisch modifiziert ist oder mit Zuckerketten verknüpft ist,
als wirksamer Bestandteil zur Herstellung einer vor Strahlung schützenden Zusammensetzung.

2. Verwendung eines Polypeptids mit humaner ADF-Aktivität nach Anspruch 1 als wirksamer Bestandteil zur Herstellung einer entzündungshemmenden Zusammensetzung.

3. Verwendung eines Polypeptids mit humaner ADF-Aktivität nach Anspruch 1 als ein wirksamer Bestandteil zur Herstellung einer Zusammensetzung zur Behandlung von Rheumatismus.

4. Verwendung eines Polypeptids mit humaner ADF-Aktivität nach Anspruch 1 als ein wirksamer Bestandteil zur Herstellung einer Zusammensetzung zur Behandlung von Autoimmunkrankheiten.

5. Verwendung eines Polypeptids mit humaner ADF-Aktivität nach Anspruch 1 als ein wirksamer Bestandteil zur Herstellung einer Zusammensetzung zur Behandlung von durch Ischämie hervorgerufenen Organschäden.

6. Verwendung eines Polypeptids mit humaner ADF-Aktivität nach Anspruch 1 als ein wirksamer Bestandteil zur Herstellung einer Zusammensetzung zur Behandlung von Arzneimittel-Toxizität.

7. Verwendung eines Polypeptids mit humaner ADF-Aktivität nach Anspruch 1 als ein wirksamer Bestandteil zur Herstellung einer Zusammensetzung zur Behandlung von Arteriosklerose.

8. Verwendung nach einem der Ansprüche 1 bis 7, worin das als ein wirksamer Bestandteil verwendete humane ADF in Escherichi coli hergestellt wird.

## Revendications

1. Utilisation d'un polypeptide ayant une activité d'ADF humain, sélectionné parmi les polypeptides (a) à (g) suivants :
(a) 
1
Val-Lys-Gln-Ile-Glu-Ser-Lys-Thr-Ala-Phe-Gln-
Glu-Ala-Leu-Asp-Ala-Ala-Gly-Asp-Lys-Leu-Val-
Val-Val-Asp-Phe-Ser-Ala-Thr-Trp-Cys-Gly-Pro-
Cys-Lys-Met-Ile-Lys-Pro-Phe-Phe-His-Ser-Leu-
50
Ser-Glu-Lys-Tyr-Ser-Asn-Val-Ile-Phe-Leu-Glu-
Val-Asp-Val-Asp-Asp-Cys-Gln-Asp-Val-Ala-Ser-
Glu-Cys-Glu-Val-Lys-Cys-Met-Pro-Thr-Phe-Gln-
Phe-Phe-Lys-Lys-Gly-Gln-Lys-Val-Gly-Glu-Phe-
Ser-Gly-Ala-Asn-Lys-Glu-Lys-Leu-Glu-Ala-Thr-
100
Ile-Asn-Glu-Leu-Val.
(b) Polypeptide déficitaire en un ou plusieurs aminoacides par rapport à (a);
(c) polypeptide dans lequel on a remplacé un ou plusieurs aminoacides par rapport à (a);
(d) polypeptide de fusion comprenant un polypeptide selon (a), (b) ou (c) et des aminoacides additionnels, dans lequel les aminoacides additionnels liés n'interfèrent pas avec l'activité biologique d'ADF ou sont susceptibles d'être facilement éliminés ;
(e) polypeptide qui est un dérivé allélique d'un polypeptide selon (a), (b), (c) ou (d);
(f) polypeptide dans lequel des aminoacides additionnels sont insérés dans la séquence d'aminoacides (a) ou (b) et
(g) polypeptide présentant une quelconque des séquences d'aminoacides (a) à (f), chimiquement modifiée ou à laquelle sont liées des chaînes d'oses,
en tant que principe actif pour préparer une composition radioprotectrice.

2. Utilisation d'un polypeptide avant une activité d'ADF humain selon la revendication 1, en tant que principe actif pour préparer une composition anti-inflammatoire.

3. Utilisation d'un polypeptide ayant une activité d'ADF humain selon la revendication 1, en tant que principe actif pour préparer une composition destinée au traitement des rhumatismes.

4. Utilisation d'un polypeptide ayant une activité d'ADF humain selon la revendication 1, en tant que principe actif pour préparer une composition destinée au traitement des maladies auto-immunes.

5. Utilisation d'un polypeptide ayant une activité d'ADF humain selon la revendication 1, en tant que principe actif pour préparer une composition destinée au traitement de lésions ischémiques organiques.

6. Utilisation d'un polypeptide avant une activité d'ADF humain selon la revendication 1, comme principe actif pour préparer une composition destinée au traitement de la toxicité médicamenteuse.

7. Utilisation d'un polypeptide avant une activité d'ADF humain selon la revendication 1, comme principe actif pour préparer une composition destinée au traitement de l'artériosclérose.

8. Utilisation selon une quelconque des revendications 1 à 7, dans laquelle l'ADF humain utilisé comme principe actif est produit dans Escherichia coli.
